# EUROPEAN PATENT SPECIFICATION

(11) **EP 3 797 684 B1**
(45) Date of publication and mention of the grant of the patent: **17.06.2026**
(21) Application number: 20196802.1
(22) Date of filing: 18.09.2020
(51) Int. Cl.: A61B 5/1495, A61B 5/11

(54) **PROGRAM FOR ANALYZING MEASUREMENT DATA OF MUSCLE ACTION POTENTIALS**
PROGRAMM ZUR DATENANALYSE VON MUSKELAKTIONSPOTENTIALEN
PROGRAMME D'ANALYSE DES DONNÉES DE MESURE DES POTENTIELS D'ACTION MUSCULAIRE

(30) Priority: 30.09.2019 JP 2019179903
(43) Date of publication of application: 31.03.2021
(73) Proprietor: GC Corporation, Sunto-gun, Shizuoka 410-1307 (JP)
(72) Inventor: TSUCHIYA, Ryoji, Tokyo, 174-8585 (JP); YANO, Seiji, Tokyo, 174-8585 (JP)
(74) Representative: Müller-Boré & Partner Patentanwälte PartG mbB

(56) References cited:
- EP-A1- 1 610 862
- EP-B1- 2 200 510
- JP-A- 2010 137 015
- OMOTO KATSUHIRO ET AL: "A preliminary investigation of reproducibility of EMG signals during daytime masticatory muscle activity using a portable EMG logging device", JOURNAL OF ELECTROMYOGRAPHY AND KINESIOLOGY, ELSEVIER, AMSTERDAM, NL, vol. 25, no. 4, 14 May 2015 (2015-05-14), pages 603 - 611, XP029215150, ISSN: 1050-6411, DOI: 10.1016/J.JELEKIN.2015.04.012

## Description

### FIELD

The present invention relates to a program for analyzing measurement data of muscle action potentials.

### BACKGROUND

Electromyography is known as a method of estimating a tension state of muscle. In electromyography, electric potentials generated according to tension of muscle (muscle action potentials) is measured via an electrode, which makes it possible to estimate timings a muscle within a sensing coverage of the electrode tenses at and how strong a muscle within a sensing coverage of the electrode tenses. For example, Patent Literature 1 discloses a muscle activity monitoring system including an electromyograph.

### CITATION LIST

### Patent Literature

PTL 1: JP 2010-137015 A
Document EP 1 610 862 A1 describes an apparatus for monitoring muscle activity. The apparatus comprising means for providing signals indicative of muscle activity, for example EMG-signals, means for processing of said signals in order to detect a particular activity and means for providing a feedback signal, wherein said device is designed in order to be individually adaptable in a set-up mode. The apparatus can be used for detecting and prevention of undesired activities such as bruxism, movements that are damaging or unwanted.

### SUMMARY

### Technical Problem

Waveform data obtained by means of an electromyograph contains various pieces of data of noise such as noise of alternating current of mains electricity, drift noise generated by person's respiration, body motion, etc., and noise by frequency components including artifacts like spikes. These pieces of data of noise can be removed using a differential amplifier or high-pass and low-pass filters.

When muscle action potentials of (a) masseter and/or temporal muscle(s) are measured by means of an electromyograph in order to determine whether bruxism during sleep occurs or not, more pieces of data of noise are contained than usual because of the measurements for many hours, and further an unstable contact state of an electrode and skin (off the right position by turning over during sleep etc., and adhesion of tape etc.) etc. Especially when an electromyograph with which a patient can make measurements at home or the like by oneself is used, the patient oneself is likely to turn a switch to ON and/or OFF in the state where the electromyograph is not arranged on one's face, which leads to a tendency for noise occurring just after the start of the measurements and just before the end of the measurements compared to general electromyographs, and time for noise occurring tends to vary. Such pieces of data of noise are the same kind as those of the foregoing noise, but all thereof are difficult to be removed by the foregoing way and/or working out to the device.

Particularly in bruxism measurements, the total number of bruxism occurring overnight, and the time average of the occurrence are each evaluation criteria. If pieces of data of that noise cannot be extracted, each piece of the data is added to the number as one occurrence, to affect measurement accuracy.

With the foregoing problem in view, an object of the present invention is to provide a program for analyzing measurement data of muscle action potentials which can improve measurement accuracy in bruxism measurements.

### Solution to Problem

One aspect of the present invention is a program for analyzing measurement data of a muscle action potential of bruxism from waveform data generated by means of an according to the appended claims.

The program may further comprise: displaying or automatically excluding the extracted piece of data.

The electromyograph may be arrangeable on an examinee's face for measuring bruxism.

### Advantageous Effects

The present invention makes it possible to efficiently extract pieces of data of noise generated by causes other than bruxism, from waveform data collected by means of an electromyograph etc., to improve measurement accuracy of bruxism.

### BRIEF DESCRIPTION OF DRAWINGS

Fig. 1 is a flowchart showing steps of measuring muscle action potentials for bruxism measurements.
Fig. 2 is a flowchart showing steps of S2 of obtaining data.
Fig. 3 partially shows an example of obtained waveform data.
Fig. 4 partially shows the example of obtained waveform data.
Fig. 5 partially shows the example of obtained waveform data.
Fig. 6 is a flowchart showing steps of S3 of analyzing the obtained data.
Fig. 7 shows configuration of a computer 10.

### DESCRIPTION OF EMBODIMENTS

The present invention will be described based on examples of embodiments. The present invention is defined by the appended claims.

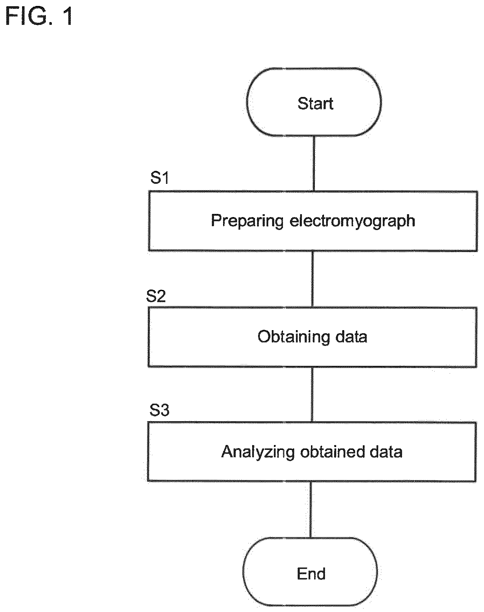

Measurements of muscle action potentials for bruxism measurements can be made by, for example, steps shown in Fig. 1: here, the steps include S1 of preparing an electromyograph, S2 of obtaining data, and S3 of analyzing the obtained data.

S1 of preparing an electromyograph is a step of preparing an electromyograph with which muscle action potentials of (a) masseter and/or temporal muscle(s) in an examinee's face for determining whether bruxism occurs or not can be measured.

The type of the electromyograph, the method of arranging the electromyograph on a face, etc. are not particularly limited, and are the same as any known ones. Among them: preferably, the electromyograph is not connected to other devices, and obtained pieces of waveform data are stored one by one in a storage medium that is attachable to and detachable from a main body thereof (such as a microSD card); and further preferably, the electromyograph is in a small size (within 70 mm × 50 mm × 20 mm). This can improve reproducibility of normal sleep during measurement, to improve accuracy of bruxism measurements. Means for arranging the electromyograph on a face is not particularly limited either, and examples thereof include pasting with adhesive double sided tape.

A small-sized electromyograph arranged independently from other members as described above tends to lead to data of noise of many kinds and a large amount. Pieces of waveform data of such noise can be effectively extracted by S3 of analyzing the obtained data of the present embodiment which will be described later.

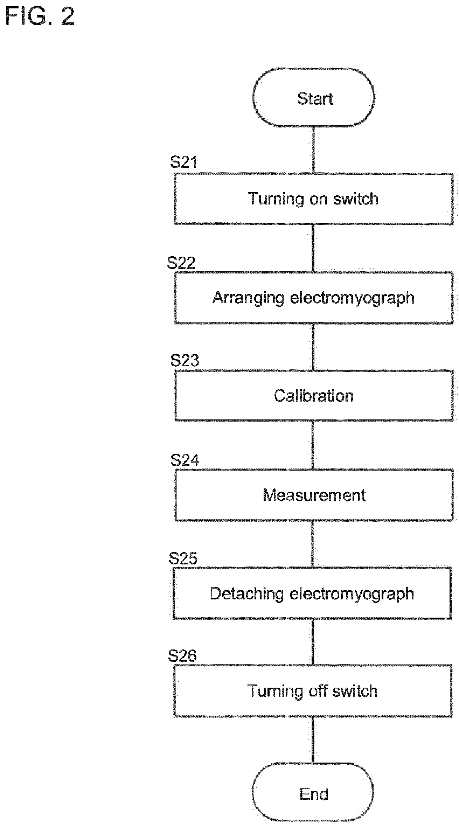

S2 of obtaining data is a step of obtaining waveform data by means of the electromyograph, and, for example, can be carried out as follows. Fig. 2 shows a flow. As is seen from Fig. 2, S2 of obtaining data includes each step of S21 of turning on a switch, S22 of arranging the electromyograph, S23 of calibration, S24 of measurement, S25 of detaching the electromyograph, and S26 of turning off the switch. Each of the steps is as follows.

S21 of turning on a switch is a step of turning on a switch of the electromyograph. In the present embodiment, collection of waveform data is started at the time point when the switch is turned on.

In S22 of arranging the electromyograph, the electromyograph with which collection of data is started in step S21 of turning on a switch is arranged on an examinee's face. Specifically, the electromyograph is arranged at a position where muscle action potentials of (a) masseter and/or temporal muscle(s) in the examinee's face for determining whether bruxism occurs or not can be measured.

S23 of calibration is a step of performing calibration action. Calibration action is (an) action(s) of a hard bite and/or tapping (repeatedly biting upper and lower teeth) etc. by an examinee. This action makes it possible to obtain waveform data of an intended hardest bite at that time. When both actions of a hard bite and tapping are performed, characteristic waveform data of these actions in combination can be obtained.

The calibration action is not limitedly performed once in a series of measurements, but may be performed a plurality of times. The calibration action is repeatedly performed, for example, when the electromyograph is attached again after coming off during the measurements, and/or before the measurements are ended.

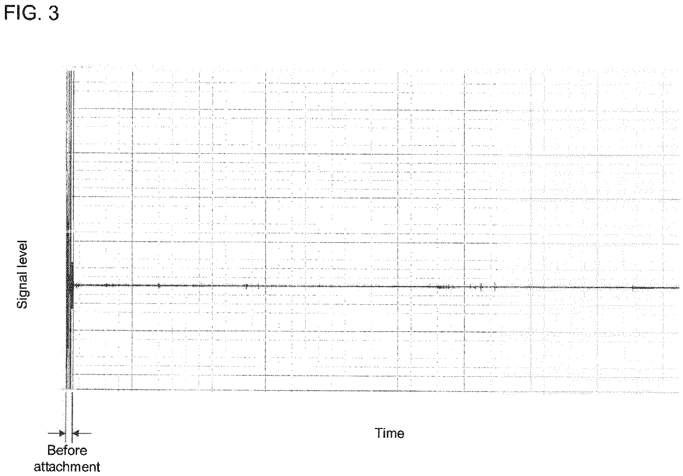

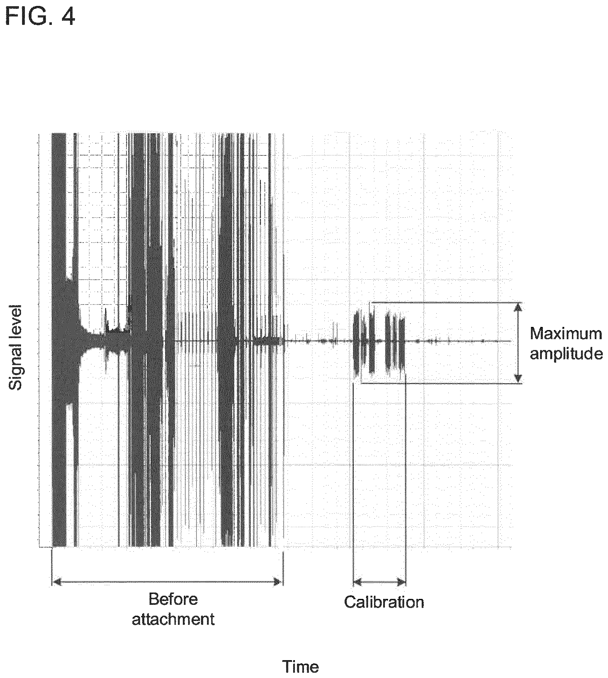

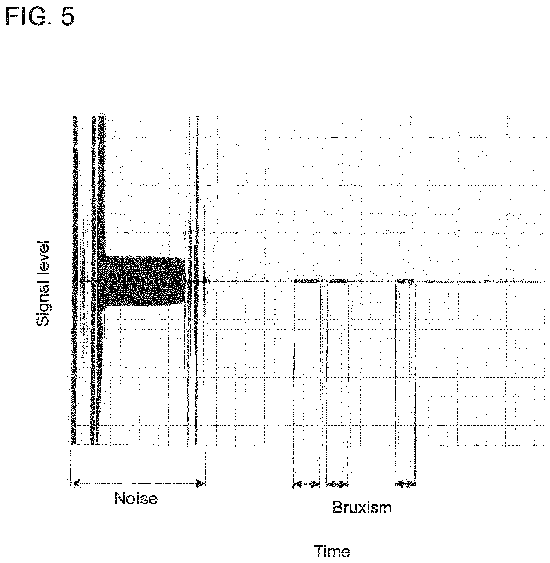

S24 of measurement is a step of measuring bruxism. Bruxism measurements usually continue overnight, and waveform data is collected without a break. Figs. 3 to 5 partially show an example of the obtained waveform data. In Figs. 3 to 5, the horizontal axis shows time, and the vertical axis shows a signal level. Figs. 4 and 5 are partially enlarged charts of the waveform data of Fig. 3. As can be seen from them, the waveform data so far contains not only a piece of waveform data in a normal state and a piece of waveform data when bruxism occurs, but also a piece of waveform data since S21 of turning on a switch until S22 of arranging the electromyograph (before attachment), a piece of waveform data of the calibration, and a piece of waveform data of noise by rubbing etc.

In S25 of detaching the electromyograph, the electromyograph is detached from the examinee's face after step S24 of measurement is ended. The electromyograph still operates at that time, to continue to collect waveform data.

S26 of turning off the switch is a step of turning off the switch of the electromyograph. The collection of waveform data is ended at this time point. The finally obtained waveform data therefore contains the foregoing pieces of data, and a piece of waveform data during S25 of detaching the electromyograph and S26 of turning off the switch.

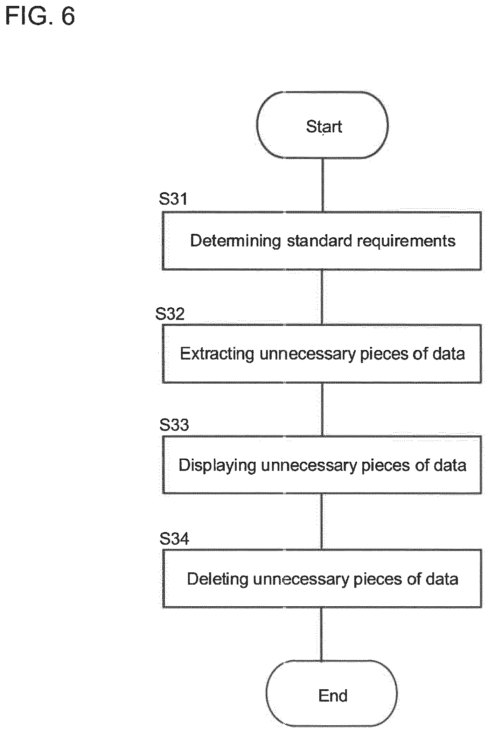

Returning to Fig. 1, S3 of analyzing the obtained data will be described. S3 of analyzing the obtained data is a step of analyzing the waveform data obtained in S2 of obtaining data, or data obtained based on this waveform data, to improve accuracy of data on bruxism. For example, S3 can be carried out as follows. Fig. 6 shows a flow. As is seen from Fig. 6, S3 of analyzing the obtained data includes S31 of determining standard requirements, S32 of extracting unnecessary pieces of data, S33 of displaying the unnecessary pieces of data, and S34 of deleting the unnecessary pieces of data. Each of the steps is as follows. "A piece of data" here means part of data in the waveform data, or the data obtained based on this waveform data.

S31 of determining standard requirements is a step of determining requirements that constitute the standard to determine necessary or unnecessary pieces of data when S32 of extracting unnecessary pieces of data, which is a step thereafter, is carried out. That is, whether a piece of data is unnecessary is determined based on the requirements determined in this S31 of determining standard requirements.

These standard requirements are not particularly limited as long as a piece of data of noise can be distinguished, and it can be distinguished that a certain piece of data is not a piece of data of noise. As shown in Fig. 4, the maximum amplitude of a piece of data derived from the intended hardest bite and/or tapping (action(s) in the calibration) in awakening can be used as a standard value. According to the invention, the maximum amplitude of a piece of data derived from the hardest bite and/or tapping recorded during sleep may be used as a standard value. A value obtained by multiplying any of these maximum amplitudes by a given coefficient may be also used as a standard value. The given coefficient is preferably at least 1, and particularly preferably 1.3 to 2.0, which is at least the ratio of the unconsciously exerted maximum muscular strength, compared to consciously exerted maximum muscular strength. A user coefficient may be changed in the range of these values. A piece of data for determining such standard requirements are extracted either manually or automatically. For example, when a bite and tapping are continuously carried out, a waveform of a piece of data thereon is characteristic, which makes it easy to automatically extract this piece of data.

Data supplied for S31 of determining standard requirements may be the waveform data itself obtained in S2 of obtaining data, and may be data conducted by prescribed processing such as average processing is performed on that waveform data. In this case, data based on the waveform data is supplied.

The maximum value (the maximum value of sensitivities) of a potential that can be measured by means of the electromyograph may be used together for the standard requirements. For example, the minimum value out of a standard value determined by the waveform data and a standard value determined by the maximum value of sensitivities can be employed for the final standard requirements.

S32 of extracting unnecessary pieces of data is a step of extracting pieces of data determined based on the standard requirements obtained in S31 of determining standard requirements, from the waveform data or the data based on the waveform data. This makes it possible to distinguish the piece of data before the electromyograph is attached, the piece of data while the electromyograph is detached and the switch is turned off, and the piece of data of noise by rubbing etc., which are unnecessary for analyzing bruxism, from the piece of data in a normal state and the piece of data when bruxism occurs, to extract them.

That is, at least a piece of data which does not meet the standard requirements obtained in S31 of determining standard requirements (such as the pieces of data shown as "before attachment" in Fig. 4 and "noise" in Fig. 5) is extracted.

As extraction of intervals of the unnecessary pieces of data, not only an interval, a piece of data in which does not meet the standard requirements, but also certain times before and after that interval (for example, 1 minute each) may be determined as intervals of the unnecessary pieces of data; and the proportion or the number of the peaks outside the standard requirements within a certain time (for example, at least 20% within one minute) may be extracted. This makes it possible to set the intervals in the measurements when accuracy may be low as unnecessary intervals. The time is preferably such a degree as not to affect the number and the time average of bruxism in the measurements for several hours during sleep (for example, within 10 minutes), and is desirably longer than the interval of a piece of data that does not meet the standard requirements (for example, at least 10 seconds). A user may change the time in this range.

An interval of a piece of data in the calibration action, and certain times before and after the calibration action may be included in the intervals of the unnecessary pieces of data since not being during sleep.

S33 of displaying the unnecessary pieces of data is a step of displaying the pieces of data extracted in S32 of extracting unnecessary pieces of data. The way of the display is not particularly limited: the extracted pieces of data are shown in a different color or different brightness from the other pieces of data on a screen that displays the waveform data or the data based on the waveform data.

S34 of deleting the unnecessary pieces of data is a step of deleting the pieces of data extracted in S33 of extracting the unnecessary pieces of data. This makes it possible to delete the unnecessary pieces of data from the waveform data or the data based on the waveform data, to improve the measurement accuracy of the bruxism measurements.

S33 of displaying the unnecessary pieces of data may be provided as necessary, and is not necessarily provided. That is, S33 of displaying the unnecessary pieces of data is necessary when an operator confirms the pieces of data extracted in S32 of extracting unnecessary pieces of data, and thereafter instructs to delete these pieces of data individually or altogether, then S34 of deleting the unnecessary pieces of data is carried out. In contrast, S33 of displaying the unnecessary pieces of data is not necessarily included when the pieces of data extracted in S32 of extracting unnecessary pieces of data is automatically deleted in S34 of deleting the unnecessary pieces of data without the display thereof.

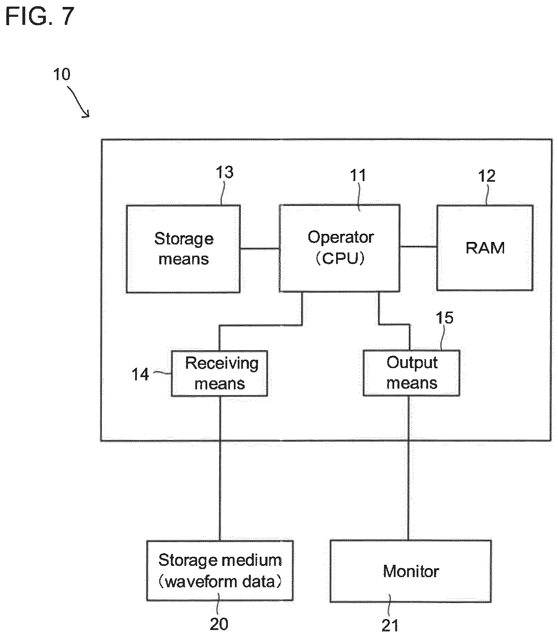

Step S3 of analyzing the obtained data to which each of those steps belongs is carried out by any of computers. That is, each step of S31 of determining standard requirements, S32 of extracting unnecessary pieces of data, S33 of displaying the unnecessary pieces of data, and S34 of deleting the unnecessary pieces of data, which are included in S3 of analyzing the obtained data, is carried out by calculating a program including steps each corresponding to the foregoing steps to output the results, by a computer. Fig. 7 shows an explanatory view. As can be seen from Fig. 7, the computer 10 includes an operator 11, RAM 12, a storage means 13, a receiving means 14, and an output means 15.

The operator 11 is configured by a so-called CPU (central operator), and is a means that can be connected to and control each of the foregoing components. The operator 11 also executes various programs stored in the storage means 13 etc. that function as storage media, to do calculations based on this as a means of generating various pieces of data and selecting data. In the present embodiment, a program including steps each corresponding to the steps included in S3 of analyzing the obtained data is stored in this storage means 13, and is calculated in the operator 11, and then the results are obtained.

The RAM 12 is a component to function as a workspace for the operator 11, and a storage means for temporary data. The RAM 12 may be configured by SRAM, DRAM, flash memory, or the like, and is the same as known RAM.

The storage means 13 is a member to function as a storage medium in which programs and data which are the bases of various calculations are stored. In the storage means 13, various intermediate and final results obtained by execution of programs may be stored. In the present embodiment, the program including each corresponding step included in S3 of analyzing the obtained data is stored in this storage means 13.

The receiving means 14 is a member connected to an attachable and detachable storage medium 20 included in the electromyograph, and having a function for taking in the waveform data stored in this storage medium.

The output means 15 is a member having a function of outputting information to be output to the outside among the results obtained by the calculations by the operator 11. In the present embodiment, a monitor 21 is connected to the output means 15, which makes it possible for an operator to see the results on the monitor 21 via a screen.

According to the computer 10, the waveform data or the data based on the waveform data is taken in via the receiving means 14 from the attachable and detachable storage medium 20 included in the electromyograph, calculations are done by the operator 11 based on a program to execute S3 of analyzing the obtained data which is stored in the storage means 13, and the results thereof are displayed on the monitor 21 via the output means 15. The waveform data may be taken into the computer 10, to create the data based on the waveform data in the computer 10.

This makes it possible for an operator to finally obtain the results of the bruxism measurements such that the unnecessary pieces of data are deleted to improve the accuracy.

### Reference Sign List

- 10: computer
- 11: operator
- 12: RAM
- 13: storage means
- 14: receiving means
- 15: output means

## Claims

1. A computer program for analyzing measurement data of a muscle action potential of bruxism from waveform data generated by means of an electromyograph, the program comprising processor-executable steps of:
(S 31) detennining a standard requirement based on a maximum amplitude of a piece of data derived from a hardest bite and/or tapping recorded during sleep, the piece of data being selected from the waveform data or data based on the waveform data; and
(S 32) extracting a piece of data which is determined based on the requirement.

2. The program according to claim 1, further comprising the steps of: (S 33) displaying or (S 34) automatically excluding the extracted piece of data.

3. The program according to claim 1 or 2, wherein the electromyograph is arrangeable on an examinee's face for measuring bruxism.

## Patentansprüche

1. Ein Computerprogramm zur Analyse von Messdaten eines Muskelaktionspotentials bei Bruxismus anhand von Wellenformdaten, die mittels eines Elektromyographen erzeugt wurden, wobei das Programm die folgenden, von einem Prozessor ausführbaren Schritte umfasst:
(S 31) Bestimmen einer Standardanforderung auf der Grundlage einer maximalen Amplitude eines Datenelements, das aus einem während des Schlafs aufgezeichneten stärksten Zubeißen und/oder Tapping abgeleitet wurde, wobei das Datenelement aus den Wellendaten oder aus auf den Wellendaten basierenden Daten ausgewählt ist; und
(S 32) Extrahieren eines Datenelements, das auf der Grundlage der Anforderung bestimmt wird.

2. Das Programm nach Anspruch 1, ferner umfassend die Schritte:
(S 33) Anzeigen oder (S 34) automatisches Ausschließen des extrahierten Datenelements.

3. Das Programm nach Anspruch 1 oder 2, wobei der Elektromyograph zur Messung von Bruxismus am Gesicht einer Untersuchungsperson anbringbar ist.

## Revendications

1. Un programme informatique pour analyser des données de mesure d'un potentiel d'action musculaire lié au bruxisme à partir de données de forme d'onde générées au moyen d'un électromyographe, le programme comprenant des étapes exécutables par processeur consistant à :
(S 31) déterminer une exigence standard sur la base d'une amplitude maximale d'un élément de données dérivé d'un serrage maximal des dents et/ou d'un *taping* dentaire enregistrés pendant le sommeil, ledit élément de données étant sélectionné parmi les données de forme d'onde ou des données basées sur les données de forme d'onde ; et à
(S 32) extraire un élément de données qui est déterminé sur la base de l'exigence.

2. Le programme d'après la revendication 1, comprenant en outre les étapes consistant à :
(S 33) afficher ou (S 34) exclure automatiquement ledit élément de données extrait.

3. Le programme d'après la revendication 1 ou 2, sachant que l'électromyographe peut être placé sur le visage d'un sujet examiné pour mesurer le bruxisme.
